# EUROPÄISCHE PATENTANMELDUNG

(11) **EP 2 123 171 A1**
(43) Veröffentlichungstag der Anmeldung: **25.11.2009**
(21) Anmeldenummer: 09450103.8
(22) Anmeldetag: 20.05.2009
(51) Int. Cl.: A23K 1/16, A23K 1/18, A61P 1/00, A61P 3/00, A61P 9/00, A61P 13/00

(54) **Futtermischung**

(30) Priorität: 20.05.2008 AT 28808 U
(71) Anmelder: Thannesberger, Otto, 4611 Buchkirchen (AT)
(72) Erfinder: Thannesberger, Otto, 4611 Buchkirchen (AT)
(74) Vertreter: Hübscher, Helmut

(57) **Zusammenfassung**

Futtermischungen zur Einhaltung unterschiedlicher, gezielter Fütterungsbedingungen für Untergruppen einer Tierart, insbesondere bei einzeln gehaltenen Haus- und Kleintieren, deren Ernährungsbedürfnisse sich in einzelnen Gruppen von den Ernährungsbedürfnissen der Tiere anderer Gruppen unterscheiden, sieht vor, dass für die Tiere jeder einzelnen Art eine gemeinsame, den größeren Anteil der Futtermischung bildende Basis und für Untergruppen je ein unterschiedlicher, die Basis für die jeweilige Tiergruppe zur fertigen Futtermischung ergänzender Zusatz vorgesehen ist. Die Basis ist als Mischung aus zwei oder insbesondere mehreren Zutaten ausgeführt und enthält für alle Tiere verträgliche Zutaten, die in der Endmischung auch für die Gruppe bzw. Gruppen von darauf empfindlichen Tieren noch eine jeweils zulässige Konzentration dieser Zutaten ergeben. Der Zusatz ist ebenfalls als Mischung ausgeführt, von dem Bestandteile im Bedarfsfall die vorhandenen Bestandteile der Basismischung auf die für die jeweilige Einzeltiergruppe vorgesehene Konzentration ergänzen und die nach einer vorgegebenen Rezeptur zusätzliche, aus Tabellen oder Plänen entnehmbare Bestandteile, sowie im Bedarfsfall der jeweiligen Einzeltiergruppe Duftstoffe und pharmakologisch wirksame Stoffe.

## Beschreibung

Die Erfindung betrifft ein Futtermittel für unterschiedliche ernährungsphysiologische Fütterungsbedingungen für Untergruppen einer Tierart, insbesondere für Hunde und Katzen, wobei sich die Ernährungsbedürfnisse der Tiere der einzelnen Untergruppen der jeweiligen Tierart beispielsweise durch unterschiedliches Alter und Krankheiten von den Ernährungsbedürfnissen anderen Untergruppen derselben Tierart unterscheiden, mit einer den größeren Anteil bildenden Basis und mit wenigstens einem ernährungsphysiologisch bedingten Zusatz.

Derartige Futtermischungen dienen zur Einhaltung unterschiedlicher,' gezielter Fütterungsbedingungen für Untergruppen einer Tierart, insbesondere bei einzeln, paarweise oder in Kleingruppen gehaltenen Haus- und Kleintieren, z. B. Katzen, Hunden und Pferden, wobei die Ernährungsbedürfnisse der Tiere der einzelnen Gruppen der jeweiligen Tierart sich z. B. durch verschiedenes Alter, Krankheiten, Fehlernährungen usw. von den Ernährungsbedürfnissen der Tiere anderer Gruppen unterscheiden.

Fertige Futtermischungen der gegenständlichen Art werden vorbereitet und in Einzelpackungen verschiedener Größe verkauft. Dabei müssen diese Futtermischungen wegen der langen Weg- und Lagerungszeiten haltbar hergestellt, verpackt und in Einzelpackungen verschiedener Größen verkauft werden, wobei viele an sich vorteilhaften Zutaten wegen ihrer Reaktion mit anderen Zutaten während der Lagerung nicht einsetzbar sind. Die Zusammensetzungen der Futtermischungen richten sich bei solchen Tieren auch bei der gleichen Art nach dem Alter und vielfach auch nach auftretenden Krankheiten oder Vorbeugungsbehandlungen gegen solche Krankheiten oder zeitweise notwendigen Diäten. Bei bestimmten Krankheiten ist jedenfalls eine Umstellung der Ernährung unerlässlich. Die Therapie erstreckt sich nicht nur auf die Medikation, sondern muss durch eine bestimmte Diät ergänzt werden. Für diese Diäten gibt es Fertigfuttermittel, die in Dosen als Feuchtdiätfuttermittel und in Säcken als Trockendiätfuttermittel entwickelt und insbesondere von den Tierärzten abgegeben werden. Die Diätfuttermittel unterscheiden sich durch eine von der normalen Ernährung deutlich abweichende Kostform als Mittel zur gezielten therapeutischen oder prophylaktischen Beeinflussung des Stoffwechsels. Beispielsweise hat ein fütterungsfertiges Herz-Diätfuttermittel, z. B. für Hunde und Katzen zur Unterstützung von deren Herzfunktion bei chronischer Insuffizienz u. a. als wichtiges Merkmal einen niedrigen Natriumgehalt und ein weites K/Na-Verhältnis. Ein Haut-Diätfuttermittel zur Unterstützung der Hautfunktion bei Dermatose und übermäßigem Haarausfall soll als wesentliches ernährungsphysiologisches Merkmal u. a. einen hohen Gehalt an essentiellen Fettsäuren aufweisen. Jungtiere benötigen auch für die normale Ernährung mehr Rohprotein als ausgewachsene Hunde, dafür aber meist einen geringeren Anteil an Kohlehydraten.

Wie schon erwähnt wurde, werden für alle diese erwähnten Fälle fertige Futtermittel nach einer festgelegten Rezeptur angeboten, was zu einer äußerst aufwendigen Produktion selbst bei den für eine Tierart bestimmten Futtermitteln führt, wozu noch kommt, das jedes neue Futtermittel einen langen Weg durch verschiedene Überprüfungen der Rezeptur und der Wirksamkeit gehen muss, ehe es tatsächlich eingesetzt werden soll.

Die gegenständlichen Futtermischungen unterscheiden sich grundsätzlich von Futtermischungen, die bei Gruppen-, insbesondere Massenhaltung, von Großtieren, z. B. Schweinen, eingesetzt werden. Solche Futtermischungen werden unmittelbar vor dem Fütterungsvorgang vorbereitet, sodass weder eine längere Haltbarkeit noch Verpackungen erforderlich werden. Meist wird mit einer bzw. zwei Gesamtrezepturen das Auslangen gefunden, wobei auch hier die exakte Zusammensetzung nicht kritisch ist. Für Schweine kann eine hier meist in flüssiger oder halbflüssiger Form zu verabreichende Futtermischung aus einer Grundlage aus Silage oder Maisprodukten bestehen, der z. B. Kraftfuttermittel und deren Mischungen sowie gegebenenfalls weitere Nähr- oder Mineralstoffe beigegeben werden. Es werden jeweils größere Mengen der Futtermischung erzeugt und zumindest einer Tiergruppe, vorzugsweise im automatischen Fütterungsverfahren, verabreicht. Für Tiere verschiedener Altersstufen und/oder vorgesehener Verwendung, z. B. als Schlacht- oder Zuchttiere, können sich die Zusammensetzungen der Beigaben und auch das Mischungsverhältnis ändern.

Aufgabe der Erfindung ist es, die Produktion der Futtermischungen der eingangs genannten Art auch für verschiedene Diät- und sonstige Spezialfuttermittel für die erwähnten Tiergruppen zu vereinfachen und trotzdem eine Futtermischung zur Verfügung stellen zu können, die genau an die jeweiligen Verwendungszwecke angepasst sind.

Die Erfindung löst diese Aufgabe dadurch, dass Basis und Futtermittelzusätze ein Futtermittelsystem bilden, bei dem eine einheitliche Basis zumindest die wesentlichen Mengen von allen Untergruppen einer Tierart gemeinen Futtermittelzutaten enthält, wobei die Konzentration zumindest eines Teils der Futtermittelzutaten in der einheitlichen Basis derart reduziert ist, dass sich diese bei vorgegebenem Mischverhältnis der einheitlichen Basis und einem auf die, insbesondere alle, ernährungsphysiologischen Bedürfnisse einer Untergruppe abgestimmten Futtermittelzusatz auf die für diese Untergruppe erforderliche Nahrungsmittelzusammensetzung ergänzt, wobei für jede Tierart die gleiche Basis und verschiedene auf die ernährungsphysiologischen Bedürfnisse der Untergruppen abgestimmte Zusätze vorgesehen sind.

Gemäß der Erfindung ist es vorgesehen, dass wenigstens ein Teil der Futtermittelzutaten, insbesondere wenigstens fünf derer, eine einheitliche Futtermittelbasis für eine Tierart bildet. Diese Basis wird, um ein vollwertiges Futtermittel für die verschiedenen Lebenslagen zu schaffen vor einem verabreichen um eine auf die jeweilige Lebensphase bzw. die jeweiligen ernährungsphysiologischen Bedürfnisse abgestimmten Futtermittelzusatz ergänzt, womit dann ein Vollwertfutter geschaffen wird. Dadurch, dass für die Tiere jeder einzelnen Art eine gemeinsame, den größeren Anteil der Futtermischung bildende Basis und für Untergruppen je ein unterschiedlicher, die Basis für die jeweilige Tiergruppe zur fertigen Futtermischung ergänzender Zusatz vorgesehen ist, lässt sich in einfacher Weise ein als Baukasten ausgebildetes hochflexibles Futtermittelsystem schaffen. Die gemeinsame Basis ist als Mischung aus zwei oder insbesondere mehreren Zutaten mit der Maßgabe ausgeführt, dass sie nur für alle Tiere der jeweiligen Tierart verträgliche Zutaten enthält und die Anteile der Zutaten höchstens gleich, vorzugsweise aber niedriger als jene Werte bemessen sind, die in der Endmischung auch für die Gruppe bzw. Gruppen von darauf empfindlichen Tieren noch eine jeweils zulässige Konzentration dieser Anteile ergeben. Der Futtermittelzusatz ist ebenfalls als Mischung ausgeführt, von dem Bestandteile im Bedarfsfall die vorhandenen Bestandteile in der Basismischung auf die für die jeweilige Einzeltiergruppe vorgesehene Konzentration ergänzen. Der Futtermittelzusatz kann die nach einer vorgegebenen Rezeptur zusätzlichen, zum Beispiel aus Fütterungstabellen, Therapieplänen oder Diätplänen entnehmbaren Bestandteile, gegebenenfalls auch Duftstoffe und pharmakologisch wirksame Stoffe je nach den Erfordernissen für diese Untergruppe enthalten.

Der Erfindungsgedanke besteht im Wesentlichen darin, dass für die Tiere jeder einzelnen Art eine gemeinsame, den größeren Anteil der Futtermischung bildende Basis und für Untergruppen je ein unterschiedlicher, die Basis für die jeweilige Tiergruppe zur fertigen Futtermischung ergänzender Zusatz vorgesehen ist, wobei die gemeinsame Basis als Mischung aus zwei oder insbesondere mehreren Zutaten mit der Maßgabe ausgeführt ist, dass sie nur für alle Tiere der verschiedenen Gruppen dieser Art verträgliche Zutaten enthält und die Anteile der Zutaten höchstens gleich, vorzugsweise aber niedriger als jene Werte bemessen sind, die in der Endmischung auch für die Gruppe bzw. Gruppen von darauf empfindlichen Tieren noch eine jeweils zulässige Konzentration dieser Anteile ergeben und dass der Zusatz ebenfalls als Mischung ausgeführt ist, von dem Bestandteile im Bedarfsfall die vorhandenen Bestandteile der Basismischung auf die für die jeweilige Einzeltiergruppe vorgesehene Konzentration ergänzen

Die normalerweise den größeren Bestandteil der Futtermischung bildenden Basis enthält alle Zutaten, die für alle Tiere der jeweiligen Tierart geeignet sind, wobei aber die Dosierung dieser Zutaten und die Beigabe weiterer Zutaten für den jeweiligen Verwendungszweck (verschiedene Diäten) variieren kann, was durch entsprechende Zusammensetzung des beispielsweise in einem variablen Mischungsverhältnis von beispielsweise 4 : 1 der Basis beigegebenen Futtermittelzusatzes erreicht wird. Es braucht also bei einer notwendigen ernährungsphysiologischen Anpassung oder neuen Anwendungsform der Futtermischung nicht jeweils das gesamte Diät-, Arznei- oder Alleinfuttermittel neu formuliert und getestet werden, sondern es ist nur notwendig, den jeweiligen Zusatz zu adaptieren oder neu zu formulieren. Damit besteht in der Produktion die Möglichkeit, für alle diese Anwendungsfälle die gleiche, nur einmal zu testende Basis zu verwenden und nur die Zusätze neu zu formulieren und allenfalls auszutesten.

Grundsätzlich lässt sich der Erfindungsgedanke für alle Futtermischungen der gegenständlichen Art mit einer größeren Anzahl von Zutaten realisieren, wobei selbstverständlich jeweils das artgerechte Futter für die jeweilige Tierart einzusetzen ist. Es können auch Futtermischungen, z. B. für andere Warmblütler z. B. Nagetiere (Hamster), Exoten, Raubtiere, Amphibien, Reptile nach den angegebenen Regeln formuliert werden.

In der Praxis kann die Basis und der Zusatz wahlweise trockene, flüssige, gelartige oder feuchte Konsistenz aufweisen, wobei nach den Einsatzbedingungen wahlweise eine Basis mit einem Zusatz gleicher oder unterschiedlicher Konsistenz zur Futtermischung zusammenführbar sind.

Die Erfindung bietet auch die Möglichkeit, alterungsempfindliche oder im Zusammenwirken mit den übrigen Futterbestandteilen nur bedingt lagerfähige Zutaten einzusetzen. Zu diesem Zweck sind für Basis und Zusatz einer Futtermischung gesonderte Vorratsräume, z. B. in Verpackungen oder Versandbehältern vorgesehen, sodass die Mischung erst vor ihrer Ausgabe fertigstellbar ist.

Lediglich als Beispiel für eine mögliche Anwendung des Erfindungsgedankens bei einer Futtermischung für Hunde kann vorgesehen werden, dass in der Basis wenigstens die Anteile der wichtigsten Zutaten z. B. an Rohprotein, Phosphor, Natrium, Kalium, Magnesium und vorzugsweise auch Rohfett auf jene Werte eingestellt sind, die durch entsprechende Anteile im Zusatz bzw. durch deren Fehlen im Zusatz in der Gesamtmischung die für den Anwendungsfall erwünschte Konzentration, z. B. eine niedrige Konzentration von Rohprotein in der Größenordnung 15 % für nierenkranke Hunde und eine entsprechend höhere Konzentration für gesunde ausgewachsene Hunde in der Größenordnung von 20 % ergeben.

Futtermischungen für Katzen und Hunde sollen sich gemäß der Erfindung von im Handel befindlichen Normal- und Diätfuttermittel grundsätzlich dadurch unterscheiden, dass die Basis
1. einen stark reduzierten Rohproteingehalt
2. einen stark reduzierten Phosphorgehalt
3. einen stark reduzierten Natriumgehalt
4. einen moderaten Fettgehalt aufweist und
5. für die Herstellung ausschließlich hypoallergene Protein- und Kohlehydratquellen Verwendung finden.

Durch die erwähnten Rezepturabweichungen der Basis von bekannten Futtermitteln kann diese universell bei allen Erkrankungsformen und für jeden Ernährungszweck eingesetzt werden. Die Basis soll jedoch nicht als Alleinfuttermittel, sondern nur mit dem jeweiligen Zusatz für den Anwendungsfall eingesetzt werden.

Zu näheren Erläuterung des Erfindungsgegenstandes wird eine mögliche Verwirklichung der Futtermischung aus der Basis und dem Zusatz für verschiedene Anwendungsfälle bei herzkranken Hunden, nierenkranke Hunde und bei der vorgesehenen Verwendung als Futtermittel für ausgewachsene gesunde Hunde angeführt. Die angegebenen Werte sind in (Gew.) Prozentanteilen der Trockensubstanz in der Basis und ebenfalls in (Gew.) Prozentanteilen der Trockensubstanz im Zusatz angeführt. Es ist vorgesehen, die Basis jeweils im Verhältnis von 3 : 1 bis 6 : 1, insbesondere 4 : 1 mit dem Futtermittelzusatz zu mischen. Die Mischung kann Volums- oder Gewichtsabhängig erfolgen. Als Mischungsbeispiel erhält ein 50 kg schwerer Hund eine tägliche Futtermenge von 625 g aus 500 g Basis und 125 g Zusatz.

Die im folgenden angegebenen Mengen der Nahrungsmittelbestandteile verstehen sich mit einer Schwankungsbreite von ±20%, vorzugsweise ±10%.

### Beispiel 1:

### Futtermischungsbasis für Hunde und Katzen:

| | Basis f. Hunde [Gew%] | Basis f. Katzen [Gew%] |
|---|---|---|
| Rohprotein | 10 - 25, insb. 18 | 20 - 35, insb. 25 |
| Rohfett | 8 - 20, insb. 12 | 10 - 25, insb. 15 |
| Kohlehydrate | 55 | 48 |
| Rohfaser | 3 | 4, 5 |
| Feuchtigkeit | 0 | 0 |
| Kalzium | 0,6 | 0,6 |
| Phosphor | 0,1 - 0,6, insb. 0,3 | 0,2 - 0,6, insb. 0,3 |
| Natrium | 0, 03 - 0,25, insb.0, 14 | 0,1 - 0,4, insb.0,2 |
| Kalium | 0,6 | 0,4 |
| Magnesium | 0,1 | 0,05 |
| Omega 3/6 Fettsäuren | 6 | - |
| Vitamine / Mineralstoffe | 4,26 | 5,95 |

Im Folgenden sind diverse Beispiele für Futtermischungen angegeben.

### Beispiel 2:

### Futtermischung für herzkranke Hunde:

| | Basis [Gew%] | Zusatz bei Herzerkrankung [Gew%] |
|---|---|---|
| Rohprotein | 18 | 20,6 |
| Rohfett | 12 | 24 |
| Kohlehydrate | 55 | 32,2 |
| Rohfaser | 3 | 2 |
| Feuchtigkeit | 0 | |
| Kalzium | 0,6 | 1 |
| Phosphor | 0,3 | 1,8 |
| Natrium | 0,14 | 0 |
| Kalium | 0,6 | 0 |
| Magnesium | 0,1 | 0,4 |
| Omega 3/6 Fettsäuren | 6 | 8 |
| Vitamine / Mineralstoffe | 4,26 | 10 |

### Beispiel 3:

### Futtermischung für nierenkranke Hunde:

| | Basis [Gew%] | Zusatz bei Nierenerkrankung [Gew%] |
|---|---|---|
| Rohprotein | 18 | 10 |
| Rohfett | 12 | 24 |
| Kohlehydrate | 55 | 44,2 |
| Rohfaser | 3 | 2 |
| Feuchtigkeit | 0 | 0 |
| Kalzium | 0,6 | 0,4 |
| Phosphor | 0,3 | 0,2 |
| Natrium | 0,14 | 0,4 |
| Kalium | 0,6 | 0,4 |
| Magnesium | 0,1 | 0,4 |
| Omega 3/6 Fettsäuren | 6 | 8 |
| Vitamine / Mineralstoffe | 4,26 | 10 |

### Beispiel 4:

### Futtermittel für die Fütterung ausgewachsener gesunder Hunde:

| | Basis [Gew%] | Zusatz Alleinfutter [Gew%] |
|---|---|---|
| Rohprotein | 18 | 28 |
| Rohfett | 12 | 12 |
| Kohlehydrate | 55 | 3 |
| Rohfaser | 3 | 35,2 |
| Feuchtigkeit | 0 | 0 |
| Kalzium | 0,6 | 1 |
| Phosphor | 0,3 | 1,8 |
| Natrium | 0,14 | 0,4 |
| Kalium | 0,6 | 0,4 |
| Magnesium | 0,1 | 0,2 |
| Omega 3/6 Fettsäuren | 6 | 8 |
| Vitamine / Mineralstoffe | 4,26 | 10 |

### Beispiel 5:

### Futtermittel für die Fütterung von Katzen mit Magen- Darmerkrankungen:

| | Basis [Gew%] | Zusatz bei Magen-Darmerkrankungen [Gew%] |
|---|---|---|
| Rohprotein | 25 | 35 |
| Rohfett | 15 | 10 |
| Kohlehydrate | 48 | 27 |
| Rohfaser | 4,5 | 17 |
| Feuchtigkeit | 0 | - |
| Kalzium | 0,6 | 1,6 |
| Phosphor | 0,3 | 1,2 |
| Natrium | 0,2 | 0,6 |
| Kalium | 0,4 | 1,8 |
| Magnesium | 0,05 | 0,1 |
| Omega 3/6 Fettsäuren | - | - |
| Vitamine / Mineralstoffe | 5,95 | 5,7 |

### Beispiel 6:

### Futtermittel für die Fütterung von Katzen mit Nierenfunktionsstörungen:

| | Basis [Gew%] | Zusatz bei Nierenfunktionsstörungen [Gew%%] |
|---|---|---|
| Rohprotein | 25 | 25 |
| Rohfett | 15 | 25 |
| Kohlehydrate | 48 | 27 |
| Rohfaser | 4,5 | 14 |
| Feuchtigkeit | 0 | - |
| Kalzium | 0,6 | 1,6 |
| Phosphor | 0,3 | 0,7 |
| Natrium | 0,2 | 0,4 |
| Kalium | 0,4 | 1,2 |
| Magnesium | 0,05 | 0,15 |
| Omega 3/6 Fettsäuren | - | - |
| Vitamine / Mineralstoffe | 5,95 | 4,95 |

### Beispiel 7:

### Futtermittel für die Fütterung von Katzen mit Neigung zu Steinbildung in den ableitenden Hamwegen:

| | Basis [Gew%] | Zusatz bei Harnsteinbildung [Gew%] |
|---|---|---|
| Rohprotein | 25 | 40 |
| Rohfett | 15 | 20 |
| Kohlehydrate | 48 | 12 |
| Rohfaser | 4,5 | 15 |
| Feuchtigkeit | 0 | - |
| Kalzium | 0,6 | 1,6 |
| Phosphor | 0,3 | 2 |
| Natrium | 0,2 | 0,8 |
| Kalium | 0,4 | 2 |
| Magnesium | 0,05 | 0,05 |
| Omega 3/6 Fettsäuren | - | - |
| Vitamine / Mineralstoffe | 5,95 | 6,55 |

## Patentansprüche

1. Futtermittel für unterschiedliche ernährungsphysiologische Fütterungsbedingungen für Untergruppen einer Tierart, insbesondere für Hunde und Katzen, wobei sich die Ernährungsbedürfnisse der Tiere der einzelnen Untergruppen der jeweiligen Tierart beispielsweise durch unterschiedliches Alter und Krankheiten von den Ernährungsbedürfnissen anderen Untergruppen derselben Tierart unterscheiden, mit einer den größeren Anteil bildenden Basis und mit wenigstens einem ernährungsphysiologisch bedingten Zusatz, **dadurch gekennzeichnet, dass** Basis und Futtermittelzusätze ein Futtermittelsystem bilden, bei dem eine einheitliche Basis zumindest die wesentlichen Mengen von allen Untergruppen einer Tierart gemeinen Futtermittelzutaten enthält, wobei die Konzentration zumindest eines Teils der Futtermittelzutaten in der einheitlichen Basis derart reduziert ist, dass sich diese bei vorgegebenem Mischverhältnis der einheitlichen Basis und einem auf die ernährungsphysiologischen Bedürfnisse einer Untergruppe abgestimmten Futtermittelzusatz auf die für diese Untergruppe erforderliche Nahrungsmittelzusammensetzung ergänzt, wobei für jede Tierart die gleiche Basis und verschiedene auf die ernährungsphysiologischen Bedürfnisse der Untergruppen abgestimmte Zusätze vorgesehen sind.

2. Futtermittelsystem zum individuellen Herstellen eines Futtermittels nach Anspruch 1, **dadurch gekennzeichnet, dass** Basis und Futtermittelzusätze zu individuellen Verkaufseinheiten abgefüllt sind.

3. Futtermischungssystem nach Anspruch 1 oder 2, **dadurch gekennzeichnet, dass** die Basis und die Futtermittelzusätze wahlweise trockene, flüssige, gelartige oder feuchte Konsistenz aufweisen, wobei nach den Einsatzbedingungen wahlweise eine Basis mit einem Futtermittelzusätze gleicher oder unterschiedlicher Konsistenz zur Futtermischung zusammenführbar sind.

4. Futtermischungssystem nach einem der Ansprüche 1 bis 3, **dadurch gekennzeichnet, dass** für die Basis und den jeweiligen Futtermittelzusatz einer Futtermischung gesonderte Vorratsräume in Verpackungen oder Versandbehältern vorgesehen sind, sodass die Mischung erst vor ihrer Ausgabe fertigstellbar ist.

5. Futtermischungssystem nach einem der Ansprüche 1 bis 4 für Hunde oder Katzen, **dadurch gekennzeichnet, dass** in der Basis wenigstens die Anteile der wichtigsten Zutaten z. B. an Rohprotein, Phosphor, Natrium, Kalium, Magnesium und vorzugsweise auch Rohfett auf jene Werte eingestellt sind, die durch entsprechende Anteile im jeweiligen Futtermittelzusatz bzw. durch deren Fehlen im Futtermittelzusatz in der Gesamtmischung die für den Anwendungsfall erwünschte Konzentration, beispielsweise eine niedrige Konzentration von Rohprotein in der Größenordnung 15 % für nierenkranke Hunde und eine entsprechend höhere Konzentration für gesunde ausgewachsene Hunde in der Größenordnung von 20 % ergeben.

6. Futtermischungssystem nach einem der Ansprüche 1 bis 5 für Hunde, **dadurch gekennzeichnet, dass** die Basis 10 bis 25 vorzugsweise 18 Gew% Rohprotein, 8 bis 20 vorzugsweise 12 Gew% Rohfett, 55 Gew% Kohlenhydrate, 3 Gew% Rohfaser, 0,6 Gew% Kalzium, 0,1 bis 0,6 vorzugsweise 0,3 Gew% Phosphor, 0,03 bis 0,25 vorzugsweise 0,14 Gew% Natrium, 0,6 Gew% Kalium, 0,1 Gew% Magnesium, 6 Gew% Omega 3/6 Fettsäuren und 4,26 Gew% Vitamine / Mineralstoffe gegebenenfalls mit einer Schwankungsbreite von ±20%, vorzugsweise ±10%, umfasst.

7. Futtermischungssystem nach einem der Ansprüche 1 bis 5 für Katzen, **dadurch gekennzeichnet, dass** die Basis 20 bis 35 vorzugsweise 25 Gew% Rohprotein, 10 bis 25 vorzugsweise 15 Gew% Rohfett, 48 Gew% Kohlenhydrate, 4,5 Gew% Rohfaser, 0,6 Gew% Kalzium, 0,2 bis 0,6 vorzugsweise 0,3 Gew% Phosphor, 0,1 bis 0,4 vorzugsweise 0,2 Gew% Natrium, 0,4 Gew% Kalium, 0,05 Gew% Magnesium und 5,95 Gew% Vitamine / Mineralstoffe gegebenenfalls mit einer Schwankungsbreite von ±20%, vorzugsweise ±10%, umfasst.

8. Futtermischungssystem nach einem der Ansprüche 1 bis 7, **dadurch gekennzeichnet, dass** das Mischungsverhältnis zwischen Basis und Futtermittelzusatz im Bereich zwischen 3 zu 1 und 6 zu 1, insbesondere 4 zu 1 Teilen, ist.
